# EUROPÄISCHE PATENTANMELDUNG

(11) **EP 0 653 213 A2**
(43) Veröffentlichungstag der Anmeldung: **17.05.1995**
(21) Anmeldenummer: 94117643.0
(22) Anmeldetag: 09.11.1994
(51) Int. Cl.: A61L 11/00

(54) **Verfahren und Vorrichtung zur Desinfektion von Abfällen**

(30) Priorität: 12.11.1993 DE 4338710
(71) Anmelder: Seifried, Dietmar, D-78606 Seitingen (DE)
(72) Erfinder: Seifried, Dietmar, D-78606 Seitingen (DE)
(74) Vertreter: Neymeyer, Franz, Dipl.-Ing. (FH)

(57) **Zusammenfassung**

Um die Desinfektion von Abfällen, insbesondere klinischen Abfällen, bei dem die Abfälle in durch abnehmbare Behälterdeckel (62) dicht verschlossenen Abfallbehältern (9) einer Erhitzung ausgesetzt werden, einfach und sicher durchführen zu können sieht das dazu entwickelte Verfahren vor, daß in einen oder gleichzeitig in mehrere Abfallbehälter (9), die Abfälle enthalten und die durch abnehmbare Behälterdeckel (62) keimdicht versclossen sind, aus einem Dampfkessel (3) von unten überhitzter Wasserdampf von wenigstens 130°C zugeführt wird. Dabei erfolgt diese Dampfzufuhr über eine vorwählbare Behandlungsdauer, die erst zu laufen beginnt, wenn im oberen Bereich des Abfallbehälters bzw. aller Abfallbehälter (9) eine vorbestimmte Innentemperatur von wenigstens 110°C erreicht ist. Vorzugsweise beträgt die Temperatur des injizierten Wasserdampfes zwischen 130°C und 200°C.

## Beschreibung

Verfahren zur Desinfektion von Abfällen, insbesondere klinischen Abfällen, bei dem die Abfälle zur Vernichtung der Keime und Bakterien in durch abnehmbare Deckel dicht verschlossenen Abfallbehältern einer Erhitzung ausgesetzt werden.

Es ist bereits ein Verfahren zum Sterilisieren von Krankenhausmüll bekannt (DE 39 24 744 C1) bei dem mit dem genannten Krankenhausmüll gefüllte und mit einem Deckel hermetisch verschlossene Müllbehälter aus Kunststoff auf ein Vorbereitungsförderband oder einen Vorbereitungsdrehtisch in eine Schleusenkammer eines Sterilisierungstunnels gebracht werden, die danach hermetisch am Eingabeende verschlossen wird. Innerhalb des Sterilisierungstunnels wird dann eine Schleusentür geöffnet durch welche die Müllbehälter von dem Vorbereitungsförderband bzw. Vorbereitungsdrehtisch der Schleusenkammer auf ein Hauptförderband oder einen Hauptdrehtisch innerhalb einer Sterilisierungskammer überführt werden, die danach hermetisch verschlossen wird. Über beweglich in der Sterilisierungskammer angeordnete Injektionsnadeln wird Wasser oder Desinfektionsmittel in jeden einzelnen Müllbehälter injiziert, um den Müll zu befeuchten. Danach wird dann der befeuchtete Müll in den Müllbehältern einer Mikrowellenbehandlung unterzogen, nach deren Abschluß alle in der Sterilisierungskammer vorhandenen Müllbehälter an deren Austragsende wieder entnommen werden, wobei während dieses Entladevorgangs die Schleusentür zur Schleusenkammer, in welcher sich das Vorbereitungsförderband bzw. ein Vorbereitungsdrehtisch befindet, hermetisch verschlossen gehalten wird.

Abgesehen davon, daß die dazu erforderliche Ablage mit einem Vorbereitungsförderband und einem Hauptförderband bzw. einem Vorbereitungsdrehtisch und einem Hauptdrehtisch, die jeweils in verschiedenen, voneinander hermetisch abtrennbaren Kammern eines Sterilisierungstunnels angeordnet sind, aufwendig und teuer ist, benötigt sie auch sehr viel Platz. Hinzu kommt, daß nicht mit letzter Sicherheit vermieden werden kann, daß durch die Einstiche der Injektionsnadeln während der Mikrowellenbehandlung mit Krankheitskeimen- oder Krankheitserregern beladene Gase in die Sterilisierungskammer und nach deren entnahmeseitigem Öffnen auch ins Freie gelangen können. Durch die Mikrowellenbehandlung entsteht in den einzelnen Müllbehältern infolge der Erwärmung ein Überdruck, durch den die Gefahr eines solchen Entweichens keimbelasteter Gase sehr groß wird. Der Hauptnachteil der Mikrowellenbehandlung besteht jedoch darin, daß die Müllbehälter aus einem für Mikrowellen durchlässigen Stoff bestehen müssen, also nicht aus Metall bestehen können. Deshalb sieht dieses Verfahren auch vor, Müllbehälter aus Kunststoff zu verwenden und sie der Einfachheit halber als Einwegbehälter zu benutzen, die beispielsweise mit Hilfe eines Preßmüllcontainers entsorgt werden müssen. Das bedeutet, daß durch die Behälter selbst zusätzlicher Müll erzeugt wird, was unbedingt zu vermeiden ist. Schon aus diesem Grunde ist dieses bekannte Verfahren untauglich.

Aufgabe der Erfindung ist es, ein Verfahren und eine Vorrichtung zur Desinfektion von mit Krankheitskeimen oder -erregern verseuchter Abfällen zu schaffen, das bzw. die sicher und zuverlässig arbeitet, einfach durchzuführen und zu handhaben ist, und durch welches bzw. welche sichergestellt ist, daß den einmal geschlossenen Abfallbehältern sowohl vor als auch während des Desinfektionsprozesses keine Krankheitskeime bzw. -erreger ins Freie entweichen können.

Verfahrensmäßig wird diese Aufgabe erfindungsgemäß dadurch gelöst, daß in einen oder gleichzeitig in mehrere Abfälle enthaltende und durch abnehmbare Deckel keimdicht geschlossene Abfallbehälter aus einem Dampfkessel von unten überhitzter Wasserdampf von wenigstens 130°C zugeführt wird und daß diese Dampfzufuhr über eine vorwählbare Behandlungsdauer erfolgt, die erst zu laufen beginnt, wenn im oberen Bereich des Behälters bzw. aller Abfallbehälter eine vorbestimmte Innentemperatur von wenigstens 110°C erreicht ist.

Die erfindungsgemäß vorgesehene Vorrichtung zur Durchführung dieses Verfahrens zeichnet sich dadurch aus, daß in einem von thermischen Schutzwänden umschlossenen Behandlungsraum angeordneten, eine horizontale Abstellfläche bildenden Abstellrahmen zum Anschließen eines oder mehrerer Abfallbehälter rasterartig mehrere Dampfinjektoren angeordnet sind, welche gemeinsam über ein Ventil mit einem Dampfkessel verbunden und einzeln mit Injektorventilen der Abfallbehälter kuppelbar sind und
daß Auslaßrohrstutzen vorgesehen sind, welche die Behälterdeckel durchdringen und über eine Sammelleitung gemeinsam an einen Dampfkondensator angeschlossen, von dem das anfallende Kondensat in den Dampfkessel geleitet wird, und
daß elektrische Temperaturmelder, die jeweils durch die Behälterdeckel in die einzelnen Behälter ragen, bei Erreichen einer voreinstellbaren Betriebstemperatur ein Startsignal für eine wählbare Betriebsdauer erzeugen.

Bei dem erfindungsgemäßen Verfahren ist es möglich, Abfallbehälter aus Metall zu verwenden und so zu bauen, daß sie über eine lange Zeitdauer und sehr häufig wiederverwendet werden können, wobei sich aus hygienischen Gründen empfiehlt, die Abfallbehälter aus Edelstahl und zudem in einer stabilen Bauweise herzustellen, die eine häufige Wiederverwendung gewährleistet.

Auch bezüglich der Effektivität ist das erfindungsgemäße Verfahren dem bekannten Sterilisationsverfahren weit überlegen, weil mit dem injizierten, beispielsweise von einem Hochleistungsdampfkessel erzeugten Wasserdampf eine sowohl thermisch als auch zeitlich exakt kontrollierbare Behandlung durchgeführt werden kann, durch welche entsprechend vorgegebener Vorschriften zur Erreichung einer vollständigen Sterilisation bzw. Desinfektion des betreffenden Abfalls aus öffentlichen oder privaten Einrichtungen des Gesundheitsdienstes höchstmögliche Sicherheit gewährleistet werden kann.

Durch die Richtlinien des Bundesgesundheitsamtes und der Deutschen Gesellschaft für Krankenhaushygiene zur Prüfung von Abfalldesinfektionsverfahren auf Wirksamkeit ist es bereits bekannt, daß als Desinfektionsverfahren für Abfälle der in infrage stehenden Art ausschließlich thermische Verfahren geeignet sind bzw. daß Verfahren auf der Basis von gesättigtem Wasserdampf mit Luftaustreibung durch Evakuieren zu bevorzugen sind.

Durch das erfindungsgemäße Verfahren ist es ohne weiteres möglich sicherzustellen, daß die Krankheitserreger nicht verbreitet werden bzw. daß die Kontamination auf die ursprünglichen Gegenstände begrenzt bleibt. Dabei ist es gemäß Anspruch 2 von Vorteil, wenn die Wasserdampftemperatur zwischen 130°C und 200°C liegt, wobei der Wirkungsgrad bzw. die Desinfektion bei höheren Dampftemperaturen, also beispielsweise bei 180°C schneller vonstatten geht als bei einer in der Nähe von 130°C liegenden Dampftemperatur.

Um die Bildung von Gasblasen innerhalb der Abfälle in den einzelnen Abfallbehältern zu minimieren bzw. zu vermeiden und auch um die Behandlungsdauer verkürzen zu können, ist die Erzeugung eines Dampfüberdruckes gemäß Anspruch 3 von Vorteil.

Als einfachste Art sicherzustellen, daß die Krankheitserreger nicht verbreitet werden, ist die Verfahrensweise nach Anspruch 4 von erheblichem Vorteil, da sie sich einfach realisieren und mit absoluter Sicherheit durchführen läßt. Es besteht damit auch die Möglichkeit, den die Abfallbehälter während der Behandlungsdauer verlassenden Wasserdampf dem Dampfkessel wieder als Kondensat zuzuführen, so daß auch der Frischwasserverbrauch auf ein Minimum reduziert werden kann.

Mit Hilfe der Vorrichtung gemäß Anspruch 5 läßt sich das erfindungsgemäße Verfahren nicht nur mit der erforderlichen Betriebssicherheit sondern auch unter Gewährleistung des jeweils gewünschten Arbeitsergebnisses einer vollständigen Desinfektion bzw. Sterilisation des behandelten Abfalls durchführen, wobei die Handhabung der mit den zu behandelnden Abfällen gefüllten Abfallbehälter sehr einfach ist.

Durch die Ausgestaltung nach Anspruch 6 lassen sich sowohl die Auslaßrohrstutzen als auch die Temperaturmelder auf eine Weise in die Behälterdeckel einführen, ohne daß dabei die Gefahr besteht, daß aus den von ihren Verschlußstopfen befreiten Öffnungen der einzelnen Behälterdeckel kontaminierte Gase oder Abfallteile entweichen können. Dabei dienen die Ausgestaltungen nach Anspruch 7 und 8 zugleich zur Erhöhung dieser Sicherheit, während die Ausgestaltung nach Anspruch 9 eine sehr einfache funktionssichere Möglichkeit darstellt, die einzelnen Behälterinnenräume mit einer Sammelleitung zu verbinden, durch welche die während der Behandlungsdauer mit Wasserdampf entweichenden Gase in den geschlossenen Wasserdampfkreislauf überführt werden.

Desweiteren besteht in Verbindung mit der Ausgestaltung nach Anspruch 10 der große Vorteil, daß gleichzeitig sämtliche Behälterdeckel mit der größtmöglichen Sicherheit und der geringsten Gefahr des Entweichens von kontaminierten Gasen aus den einzelnen Behältern an die Sammelleitung angeschlossen und mit den Temperaturmeldern ausgestattet werden können.

Die gemäß Anspruch 11 vorgesehenen Verschlußstopfen stellen sehr einfache, kostengünstige Mittel dar, die in den Behälterdeckeln benötigten Öffnungen dicht zu verschließen. Außerdem lassen sie sich durch die in diese Öffnungen einzuführenden Auslaßrohrstutzen bzw. durch die Schutzrohre der Temperaturmelder leicht aus den Öffnungen der Behälterdeckel ins Behälterinnere herausstoßen. Auf diese Weise läßt sich auch sicherstellen, daß diese Öffnungen durch die auf ihrem Rand aufsitzenden Dichtungselemente der Auslaßrohrstutzen zumindest annähernd gleichzeitig mit dem Herausstoßen der Verschlußstopfen nach außen wieder abgedichtet werden und somit die Gefahr des Entweichens kontaminierter Gase auf ein Minimum reduziert ist.

Damit die aus den Deckelöffnungen herausgestoßenen Verschlußstopfen nicht verloren gehen und wieder verwendbar sind, ist es empfehlenswert, sie durch Schnüre, Ketten od. dgl. mit den Behälterdeckeln zu verbinden.

Während durch die Ausgestaltung nach Anspruch 12 das Positionieren der einzelnen Abfallbehälter auf dem Abstellrahmen und das gleichzeitige Kuppeln ihrer Injektorventile mit den Dampfinjektoren erleichtert, wird durch die gemäß Anspruch 13 auf dem Abstellrahmen angeordneten Zentrierelemente ein störungsfreies Ineingriffbringen bzw. Kuppeln der Dampfinjektoren mit den Injektorventilen der einzelnen Abfallbehälter sichergestellt. Die Zentrierelemente können aus konischen Ringen oder aus keilförmigen Fingern bestehen.

Die Ausgestaltung nach Anspruch 14 gewährleistet nicht nur eine einfache und zugleich robuste Gestaltung sowohl der Dampfinjektoren als auch der Injektorventile sondern auch eine sehr einfache Handhabung und kostengünstige Herstellung. Außerdem wird durch sie eine dichte Verbindung zwischen den Dampfinjektoren und den Injektorventilen gewährleistet.

Die Ausgestaltung nach Anspruch 15 trägt ebenfalls zur Kostensenkung bei, indem der Abstellrahmen selber als Verteiler ausgebildet ist, durch den der über eine Zuführleitung vom Dampfkessel zugeführte Wasserdampf gleichmäßig zu den einzelnen Dampfinjektoren und über diese in die einzelnen Abfallbehälter geleitet wird.

Die Ausgestaltung nach Anspruch 16, 17 und 18 hat den Vorteil, daß sich die Vorrichtung insgesamt transportieren und vorzugsweise an dem Ort, wo der zu desinfizierende Abfall anfällt, leicht aufstellen läßt, wobei der Kran gemäß Anspruch 16 und die Müllpresse gemäß Anspruch 17 ergänzende Hilfsmittel sind, welche die Benutzung der Vorrichtung und die anschließende Beseitigung der desinfizierten Abfälle zusätzlich erleichtern.

Anhand der Zeichnung wird im folgenden ein Ausführungsbeispiel der erfindungsgemäßen Vorrichtung und das mit ihr durchführbare erfindungsgemäße Verfahren näher erläutert. Es zeigt:
- Fig. 1: in schematisch vereinfachter Darstellung eine komplette Vorrichtung zur Desinfektion von Klinikabfällen in perspektivischer Seitenansicht;
- Fig. 2: in explosions-perspektivischer Ansicht die Bestandteile eines Behandlungsraumes, in dem mehrere Abfallbehälter gleichzeitig einer Desinfektions bzw. Sterilisation unterzogen werden können;
- Fig. 3: den Behandlungsraum mit zwei behandlungsbereit eingesetzten Abfallbehältern im Schnitt;
- Fig. 4: einen Abfallbehälter und einen Dampfinjektor im Schnitt;
- Fig. 5: den oberen Abschnitt eines Abfallbehälters mit verriegeltem Deckel im Schnitt;
- Fig. 6: einen Verschlußstopfen im Schnitt;
- Fig. 7: in perspektivischer Ansicht einen Dampfinjektor mit einem ihn konzentrisch umschließenden Zentrierelement des Abstellrahmens.

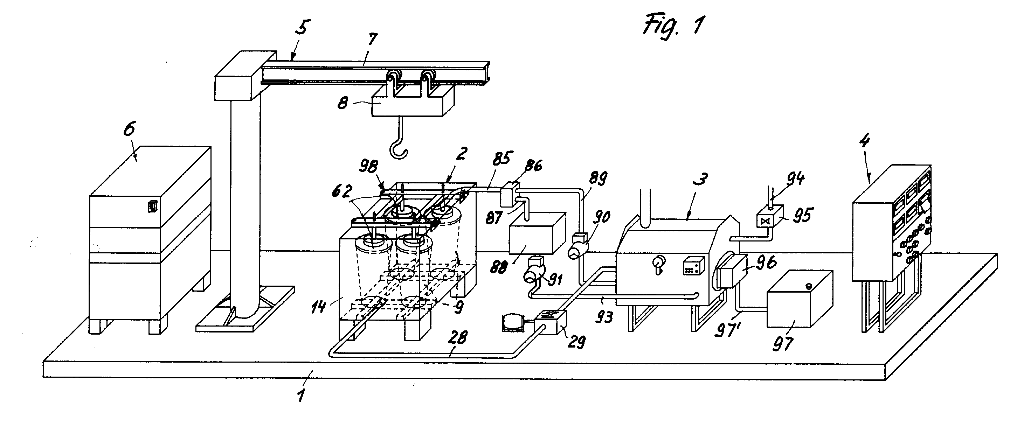

Die Fig. 1 zeigt in schematisch vereinfachter Darstellung eine komplette Vorrichtung zur Desinfektion von Abfällen, insbesondere von klinischen Abfällen, deren Bestandteile, die nachstehend im einzelnen noch näher beschrieben sind, als Gesamtanlage auf einer transportablen Konsole 1 angeordnet sind. Diese Konsole 1 kann selbst fahrbar und zu diesem Zweck mit einem Fahrgestell ausgerüstet sein. Sie kann aber auch, wie in der Fig. 1 dargestellt, lediglich als tragendes Element für die zur Anlage gehörenden Teile in Form einer großen Platte oder Palette vorgesehen sein, die auf einem Lastfahrzeug transportiert und ggf. vorort abgesetzt werden kann.

Diese Vorrichtung besteht im wesentlichen aus einem Behandlungsraum 2, einem Hochleistungsdampfkessel 3, einer elektrischen Steuereinheit 4, einem als Säulen-Schwenkkran ausgebildeten Kran 5 sowie aus einer Müllpresse 6. Der Kran 5 ist zwischen dem Behandlungsraum 2 und der Müllpresse 6 so angeordnet, daß es möglich ist, mit seiner an einem horizontalen Ausleger 7 gelagerten Laufkatze 8 und deren im einzelnen nicht dargestellten Hebezeug Müllbehälter 9 in die Behandlungskammer 2 einzusetzen, sie aus dieser herauszunehmen und in die Müllpresse 6 zu entleeren.

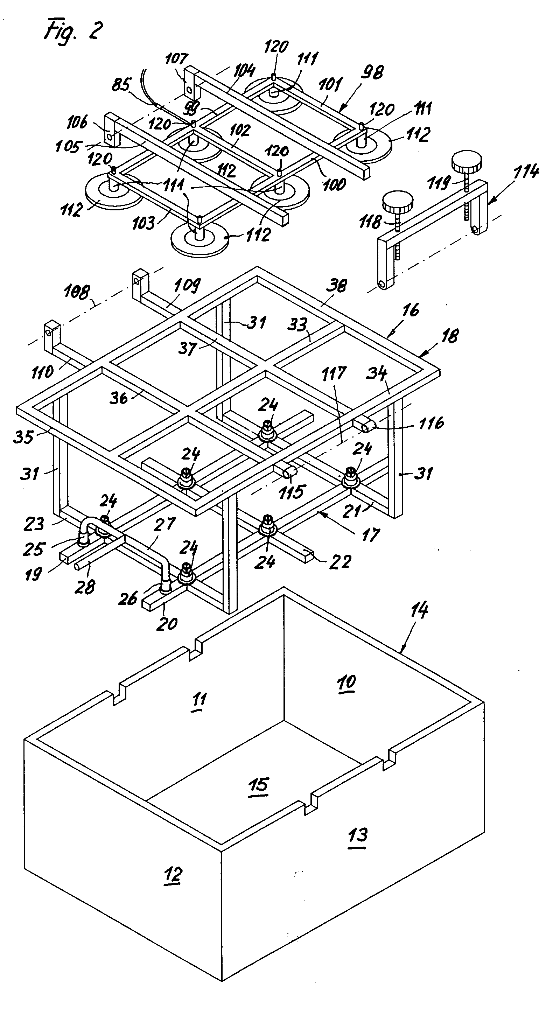

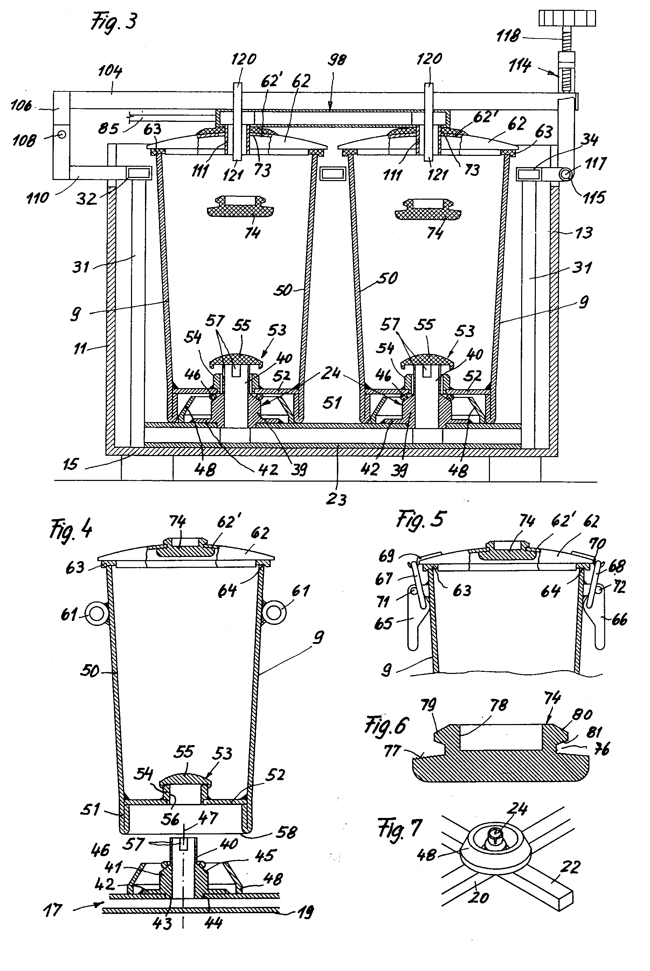

Wie aus den Fig. 2 und 3 am besten erkennbar ist, ist der Behandlungsraum 2 aus einer rechteckigen Behandlungskammer 14 gebildet, die mit vier Schutzwänden 10 bis 13 und einem horizontalen Boden 15 versehen ist. In diese Behandlungskammer 14 ist ein Rahmengestell 16 eingesetzt, das aus einem auf dem Boden 15 aufliegenden Abstellrahmen 17 und aus einem in vertikalem Abstand darüber angeordneten Aufnahmerahmen 18 besteht.

Der Abstellrahmen 17 weist zwei parallel zueinander verlaufende Längsschenkel 19 und 20 sowie drei rechtwinklig dazu verlaufende Querschenkel 21, 22 und 23 auf, die alle aus Rechteckrohren bestehen und in deren rasterartig angeordneten Kreuzungspunkten jeweils Dampfinjektoren 24 angeordnet sind, die sowohl in Längs- als auch in Querrichtung jeweils gleiche Abstände voneinander haben. Die Hohlräume der Längsschenkel 19, 20 und der Querschenkel 21, 22 und 23 sind jeweils an ihren stirnseitigen Enden verschlossen, untereinander jedoch miteinander verbunden. Die beiden Längsschenkel 19 und 20 sind durch Anschlußrohrstutzen 25 und 26 und ein Verbindungsrohr 27 mit einer Dampfzuleitung 28 verbunden, die über ein elektromotorisch betätigbares d.h. öffen- und schließbares Ventil 29 mit dem Hochleistungsdampfkessel 3 in Verbindung steht. (Fig. 1)

Der Aufnahmerahmen 18 wird von vier an den Enden der Querschenkel 21 und 23 des Abstellrahmens 17 angeordneten Vertikalstützen 31 getragen. Er besteht aus drei Längsholmen 32, 33 und 34 und vier Querholmen 35, 36, 37 und 38, die insgesamt sechs Quadrate umschließen, deren Mittelpunkte lotrecht über den Dampfinjektoren 24 des Abstellrahmens 17 liegen und deren Weite so gewählt ist, daß ein Abfallbehälter 9 mit geringem seitlichem Spiel darin seitlich geführt aufgenommen werden kann.

Wie am besten aus den Fig. 3 und 4 erkennbar ist, bestehen die Dampfinjektoren 24 jeweils aus einer zylindrischen Flanschhülse 39 mit einem im Durchmesser kleineren und dünneren oberen Abschnitt 40 und einem dickeren unteren Abschnitt 41 sowie mit einem auf dem Längsschenkel 19 bzw. 20 aufliegenden Flansch 42 und mit einem Zentrieransatz 43, der in eine Bohrung 44 des Längsschenkels 19 bzw. 20 ragt. Zwischen dem dünneren oberen Abschnitt 40 und dem dickeren, unteren Abschnitt 41 befindet sich eine konische Ringschulter 45, auf welcher ein elastischer Dichtungsring 46 aufliegt.

Die mit vertikalen Achsen 47 in die Längsschenkel 19 und 20 des Abstellrahmens 17 dicht eingesetzten Flanschhülsen 39, welche die Dampfinjektoren 24 bilden, sind von konzentrisch dazu angeordneten, sich nach oben konisch verjüngenden Zentrierelementen 48 umschlossen, die hier die Form von konischen Ringen aufweisen, die jedoch auch eine andere Form z. B. die Form von vier keilförmigen Fingern haben könnten.

Die Abfallbehälter 9 sind alle gleich ausgebildet, so daß es genügt einen einzigen in seinen Einzelteilen zu beschreiben. Ein solcher Abfallbehälter 9 besteht aus einem sich nach unten leicht konisch verjüngenden Hohlkörper 50 aus Edelstahlblech. Dieser weist an seinem unteren Ende einen zylindrischen doppelwandigen Sockelabschnitt 51 auf, an dessen oberem Ende ein ebenfalls aus Edelstahlblech bestehender Behälterboden 52 dicht eingeschweißt ist. Dieser Behälterboden 52 besitzt in seinem Zentrum ein Injektorventil 53, das aus einem dicht eingeschweißten zentrierten Rohrstutzen 54 und einer dichtend aber lose aufgesetzten Verschlußkappe 55, vorzugsweise aus leicht elastischem Kunststoff besteht. Die Bohrung 56 des Rohrstutzens 54 hat einen etwas größeren Durchmesser als der obere Abschnitt 40 der Flanschhülse 39 eines Dampfinjektors 24, so daß dieser obere Teil 40 des Dampfinjektors 24 mit radialem Spiel von unten in das Injektorventil 53 bzw. dessen Rohrstutzen 54 eingeführt werden kann. Dabei sind die axialen Längenverhältnisse so gewählt, daß beim Absenken des Abfallbehälters 9 auf den Abstellrahmen 17 und dem gleichzeitgen Einführen des oberen Abschnittes 40 eines Dampfinjektors 24 in den Rohrstutzen 54 des Injektorventils 53 die Verschlußkappe 55 vom Rohrstutzen 54 abgehoben und somit das Injektorventil 53 geöffnet wird. Am oberen Ende des Abschnittes 40 sind jeweils mehrere Schlitzöffnungen 57 vorgesehen, durch welche Wasserdampf, der über die Dampfzuleitung 28 zugeführt wird, in das Innere des ordnungsgemäß aufgesetzten Abfallbehälters 9 einströmen kann.

Um eine dichte Verbindung zwischen den Dampfinjektoren 24 einerseits und den einzelnen Injektorventilen 53 zu erhalten ist der Dichtungsring 46 so ausgebildet und angeordnet, daß er dichtend an der Unterseite des Behälterbodens 52 anliegt, wenn der Abfallbehälter 9 mit der Unterkante 58 seines Sockelabschnitts 51 auf dem Abstellrahmen 17 aufsitzt.

Es ist aus der Fig. 3 ersichtlich, daß dabei die konische Ringschulter 45 eine relativ große Toleranzbreite ermöglicht und daß das Zentrierelement 48 seine zentrierende Funktion im Zusammenwirken mit dem unteren Sockelabschnitt 51 des Abfallbehälters 9 zur sicheren Einführung des Dampfinjektors 24 in das Injektorventil 53 ausübt.

Somit ist es möglich beim Aufsetzen eines Abfallbehälters 9 durch den Aufnahmerahmen 18 hindurch auf den Abstellrahmen 17 während der Absetzbewegung eine zuverlässige und zugleich dichte Verbindung zwischen dem Innenraum des betreffenden Abfallbehälters 9 und der Wasserdampfzufuhr, d.h. eine Kupplung zwischen dem Injektorventil 53 und dem Dampfinjektor 54 herzustellen, die auf gleich einfache Weise, nämlich beim Herausheben des Abfallbehälters 9 aus dem Rahmengestell 16, wieder gelöst werden kann.

In Fig. 3 sind zwei Abfallbehälter 9 dargestellt, die auf dem Abstellrahmen 17 abgestellt sind und deren Injektorventile 53 mit den Dampfinjektoren 24 verbunden sind. Es ist dabei erkennbar, daß der Behälterboden 52 auf dem Dichtungsring 46 aufsitzt, der sich auf der konischen Ringschulter 45 des dickeren Unterteils der den Dampfinjektor 24 bildenden Flanschhülse 39 abstützt. Ebenso ist erkennbar, daß die beiden Verschlußkappen 55 vom Rohrstutzen 54 des Injektorventils 53 abgehoben sind. Man kann sich dabei auch vorstellen, daß sich diese Verschlußkappen 55 wieder schließend auf den Rohrstutzen 54 setzen können, wenn die Abfallbehälter 9 nach oben bewegt werden und die oberen Abschnitte 40 der Flanschhülsen 39 die Bohrungen 56 der Rohrstutzen 54 verlassen.

Wie aus Fig. 4 erkennbar ist, besitzt der Abfallbehälter 9 in seiner oberen Hälfte zwei sich diametral gegenüberliegende Einhängeösen 61 die sowohl als Handgriffe wie auch zum Einhängen von Kettenhaken des Hebezeugs des Krans 5 verwendet werden können.

Oberseitig ist der Abfallbehälter 9 mittels eines leicht gewölbten Deckels 62 verschlossen, der mit einem Dichtungsring 63 auf der Oberkante 64 des Hohlkörpers 50 aufsitzt und mittels zweier Spannhebel 65 und 66, die sich diametral gegenüberliegend, jedoch zu den Einhängeösen 61 versetzt am oberen Ende des Hohlkörpers 50 außenseitig angeordnet sind. Diese Spannhebel 65 und 66 sind durch zwei Ösenringe 67 bzw. 68 an Spannhaken 69 bzw. 70 des Deckels eingehängt und durch Scharniere 71 bzw. 72 schwenkbar gelagert. In Fig. 5 sind die Spannhebel 65 und 66 jeweils in ihrer Schließstellung dargestellt, in welcher der Behälterdeckel 62 mit seinem Dichtungsring 63 unter Spannung dicht auf dem oberen Rand 64 des Abfallbehälters 9 aufliegt. Solche Spannhebel 65, 66 sind an sich bekannt. Es bedarf deshalb an dieser Stelle keiner weiteren Erläuterung ihrer Funktionsweise und Handhabung.

In seiner Mitte ist der Behälterdeckel 62 mit einer zylindrischen Öffnung 73 versehen, die durch einen von unten eingesetzten Verschlußstopfen 74 dicht verschlossen ist. Dieser Verschlußstopfen 74 weist oberhalb eines Ringflansches 75 eine Ringnut 76 auf, die so gestaltet ist, daß sie den Rand der Öffnung 73 klemmend und dichtend in sich aufnehmen kann bzw. daß der Verschlußstopfen von unten so in die Öffnung 73 eingeführt werden kann, daß der Öffnungsrand dichtend in diese Ringnut 76 zu liegen kommt und daß dabei der Flansch 75 mit seiner oberen Stirnfläche 77 ebenfalls satt an der Innenseite der Deckelwandung 62' anliegt. Dieser Verschlußstopfen 74 besteht aus einem elastischen, hitzebeständigen Material, das auch Temperaturen von 200°C standhält. Um einerseits einen dichten Sitz des Verschlußstopfens 74 in der Öffnung 73 des Behälterdeckels 72 zu erhalten bedarf es einer relativ hohen Formbeständigkeit. Andererseits soll jedoch der Verschlußstopfen 74 relativ leicht und ohne Beschädigung möglichst häufig in die Öffnung 73 einsetzbar und auch wieder aus dieser herausstoßbar sein. Um diese Eigenschaft zu verbessern, ist der obere Teil des Verschlußstopfens mit einer zylindrischen Ausnehmung 78 versehen, und es ist die oberhalb der Ringnut 76 liegende, umlaufende Ringrippe 79 sowohl oberseitig als auch unterseitig jeweils mit einer konischen Fläche 80 bzw. 81 versehen.

Damit dieser Verschlußstopfen 74 nicht verlorengeht und immer wieder zur Verfügung steht, ist es zweckmäßig, ihn mittels eines nicht dargestellten Verbindungselementes, z. B. mittels einer Kette oder mittels eines anderen flexiblen Verbindungselementes an der Innenseite des Behälterdeckels 62 zu befestigen.

Nach ihrem Befüllen mit Abfällen, insbesondere mit Klinikabfällen, werden die Behälter 9 in der in Fig. 5 dargestellten Art mittels der Deckel 62 fest verschlossen, deren Öffnungen 73 durch die Verschlußstopfen 54 abgedichtet sind.

Nach dem Einsetzen der mit Abfällen gefüllten Behälter 9 in die Behandlungskammer 14, d.h. in das Rahmengestell 16, in der vorstehend beschriebenen Weise ist es auch erforderlich, ihre Innenräume an eine Sammelleitung 85 anzuschließen, durch welche sie mit einem Dampfkondensator 86 verbunden werden, der über eine Kondensatleitung 87 mit einem Kondensatauffangbehälter 88 und über eine Saugleitung 89, in der sich eine Vakuumpumpe 90 befindet, mit dem Dampfkessel 3 verbunden ist. (Fig. 1)

Auch der Kondensatauffangbehälter 88 ist durch eine Flüssigkeitspumpe 91 und eine Rückführleitung 93 so mit dem Dampfkessel 3 verbunden, daß das aufgefangene Kondensat wieder in den Dampfkreislauf des Dampfkessels 3 geführt wird, ebenso wie die durch die Vakuumpumpe 90 abgesaugten und dem Dampfkessel 3 zugeführten Gase. Die Frischwasserzufuhr zum Dampfkessel 3 erfolgt über eine Leitung 94 und ein Ventil 95. Sein Brenner 96 wird aus einem Brennstoffbehälter 97 über eine Brennstoffleitung 97' mit Brennstoff versorgt und von der Steuereinheit 4 gesteuert.

Um über die Deckel 62 sämtliche in der Behandlungskammer 14 abgestellte Abfallbehälter 9 gleichzeitig gemeinsam an die Sammelleitung 85 anschließen zu können, ist ein rechteckiger Rasterrahmen 98 vorgesehen, der zwei längs verlaufende Rahmenschenkel 99 und 100 sowie drei quer verlaufende Rahmenschenkel 101, 102 und 103 besitzt, die alle in einer gemeinsamen Ebene liegen und aus miteinander verbundenen Rechteckrohren bestehen an welche die Sammelleitung 85 gemeinsam angeschlossen ist. Mittels zweier quer verlaufender Tragschienen 104, 105 und vertikaler Winkelstücke 106, 107 ist dieser Rasterrahmen 98 um eine horizontale Gelenkachse 108 schwenkbar so an horizontalen Tragarmen 109 und 110 des Aufnahmerahmens 18 gelagert, daß er aus einer horizontalen Schließlage, die in den Fig. 2 und 3 dargestellt ist, in eine nicht dargestellte vertikale Ruhelage verschwenkt werden kann.

Dieser Rasterrahmen 98 ist in seiner Form auf den Abstellrahmen 17 abgestimmt. An ihm sind unterseitig sechs Auslaßrohrstutzen 111 so angeordnet, daß diese sich in koaxialer Lage zu und lotrecht über den Dampfinjektoren 24 des Abstellrahmens 17 befinden, wenn der Rasterrahmen 98 seine in Fig. 2 und 3 dargestelle horizontale Lage einnimmt. Wie aus Fig. 3 ersichtlich ist, ragen in dieser Schließposition des Rasterrahmens 98 seine Auslaßrohrstutzen 111 durch die Öffnungen 73 der Deckel 62 in das Innere der einzelnen Abfallbehälter 9. Dabei ist auch erkennbar, daß die Verschlußstopfen 74 sich nicht mehr in den Öffnungen 73 befinden sondern im Innern der Behälter 9. Durch das Abwärtsbewegen des Rasterrahmens 98 aus seiner hochgestellten Ruhelage in die horizontale Schließlage, haben die einzelnen Auslaßrohrstutzen 111 die Verschlußstopfen 74 der einzelnen Deckel 62 aus den Öffnungen 73 in das Behälterinnere herausgestoßen. Dadurch ist eine Verbindung zwischen den Innenräumen der einzelnen Abfallbehälter 9 und den Hohlräumen des Rasterrahmens 98 zu der Sammelleitung 85 geschaffen.

Wie aus Fig. 3 ersichtlich ist, sind die einzelnen Auslaßrohrstutzen 111 jeweils mit tellerförmigen elastischen Dichtungselementen 112 versehen, die ihre oberen Abschnitte dicht umschließen und die in dieser horizontalen Schließposition des Rasterrahmens 98 auch dichtend auf den Deckeln 62 der einzelnen Abfallbehälter 9 aufliegen, so daß durch die Öffnungen 73 weder Teile des gasförmigen noch des ggf. flüssigen oder gar festen Inhalts entweichen können. Die einzelnen Auslaßrohrstutzen 111 des Rasterrahmens 98 sind somit abgedichtet mit den Innenräumen der einzelnen Abfallbehälter 9 verbunden.

Um diese abgedichtete Anschlußverbindung gewährleisten zu können, ist ein Spannbügel 114 vorgesehen, der auf der den Tragarmen 109 und 110 gegenüberliegenden Seite des Aufnahmerahmens 18 angeordneter Scharniere 115 und 116 um eine horizontale Schwenkachse 17 schwenkbar am Rahmengestell 16 gelagert und mit zwei Spannschrauben 118 und 119 versehen ist, mit denen der Rasterrahmen 98 in der in Fig. 3 dargestellten Weise mit seinen Dichtungselementen 112 auf die Deckel 62 der einzelnen Abfallbehälter 9 gepreßt werden kann.

Zur Durchführung einer gleichzeitigen Temperaturkontrolle in den einzelnen Abfallbehältern 9, sind in den Auslaßrohrstutzen 111 des Rasterrahmens 98 jeweils in Schutzrohren 121 elektrische Temperaturmelder 120 angeordnet, die einzeln mit einer elektrischen Steuerschaltung verbunden sind und die die jeweils in den einzelnen Abfallbehältern 9 herrschenden Temperaturen an eine elektronische Temperaturüberwachungseinrichtung melden.

Diese Temperaturüberwachungseinrichtung ist auch Bestandteil der Steuereinheit 4, die überdies eine Datenregistriereinrichtung mit einem Drucker enthält, welche sämtliche für die Kontrolle und Steuerung des Desinfektionsvorganges wichtigen Daten aufnimmt, verarbeitet und festhält. Dadurch ist es möglich, die Behandlungsdaten jedes einzelnen Abfallbehälters zu sichern, um jederzeit die Kontrolle über Behandlungsintensität und Dauer zu haben und auch die Behandlungsdaten nachträglich belegen zu können.

In dem Hochleistungsdampfkessel 3 wird überhitzter Dampf mit einer Temperatur von maximal 200°C erzeugt, der in der vorstehend beschriebenen Weise den einzelnen in der Behandlungskammer 14 abgestellten Abfallbehältern 9 von unten durch das elektrisch gesteuerte Öffnen des Ventils 29 zugeführt wird. Erst wenn der letzte Temperaturmelder 120 eine vorgegebene Mindesttemperatur von beispielsweise 130°C an die Steuereinheit 4 meldet, wird eine einstellbare Zeitmesseinrichtung in Gang gesetzt, welche die vorgeschriebene Behandlungsdauer mißt und nach deren Ablauf das Signal zum Schließen des Ventils 29 und somit zur Beendigung der Dampfzufuhr aus dem Dampfkessel 3 erzeugt.

Die während der Dampfzufuhr durch die Auslaßrohrstutzen 111 und die Sammelleitung 85 in den Kondensator 86 gelangenden Gase, die mit zunehmender Zeitdauer immer mehr aus Wasserdampf bestehen, gelangen dabei in den Kondensator 86 und zum Teil über die Saugleitung 89 und die Pumpe 90 wieder in den Dampfkreislauf des Dampfkessels 3. Das im Kondensator 86 anfallende Kondensat gelangt über die Kondensatleitung 87 in den Kondensatauffangbehälter 88 und wird aus diesem von Zeit zu Zeit automatisch durch die Pumpe 91 ebenfalls dem Dampfkreislauf des Dampfkessels 3 zugeführt.

Damit ist sichergestellt, daß vom gesamten Abfallbehälterinhalt keine kontaminierten Bestandteile unkontrolliert entweichen können, d.h. es ist höchste Sicherheit gewährleistet. Hinzu kommt, daß für die Behandlung nur ein Minimum an Frischwasser benötigt wird, die Anlage also sehr rationell arbeitet.

Wichtig dabei ist, daß während der gesamten Behandlungsdauer bzw. während der gesamten Dampfzufuhr in den einzelnen Abfallbehältern 9 ein Überdruck von etwa 0,5 bis 0,8 bar herrscht, damit sich keine Gasblasen bilden können. Dieser Überdruck wird durch entsprechende Leitungsquerschnitte erzeugt.

Wie beschrieben erfolgt auch das Anschließen der einzelnen Abfallbehälter an die Dampfzufuhrleitung bzw. das Verbinden ihrer Behälterdeckel 62 mit der Sammelleitung 85 in sehr einfacher und sicherer Weise.

Auch das Entkoppeln der Abfallbehälter 9 sowohl von der Dampfzufuhr als auch von der Sammelleitung 85 geschieht auf sehr einfache Weise. Es bedarf lediglich des Entsicherns des Spannbügels 114, dessen Verschwenken nach außen und des nach oben Schwenkens des Rasterrahmens 98, um alle Behälterdeckel 62 gleichzeitig freizugeben. Die einzelnen Abfallbehälter 9 können dann einzeln mit Hilfe des Krans 5 aus der Behandlungskammer 14 entnommen und vorzugsweise gleich in die Müllpresse 6 entleert werden, um danach an ihren üblichen Befüllungsstandorten aufgestellt zu werden. Die abgenommenen Behälterdeckel 62 werden dann wieder mit Hilfe der Verschlußstopfen 74 verschlossen, so daß sie wenn der Abfallbehälter 9 wieder gefüllt ist, zum Verschließen wieder zur Verfügung stehen. Vor dem Befüllen ist es selbstverständlich notwendig, die Verschlußkappe 55 dichtend auf den Rohrstutzen 54 zu setzen, damit auch die Injektorventile 53 wieder geschlossen sind.

Vorzugsweise sind die Wände 10 bis 13 und der Boden 15 der Behandlungskammer 14 mit thermoisolierendem Material gefüllt, damit einerseits ein Berührungsschutz gegen Verbrennung und andererseits ein Schutz gegen Wärmeverluste und zugleich eine Verminderung des Kondensatanfalls innerhalb der einzelnen Abfallbehälter 9 erreicht wird.

## Patentansprüche

1. Verfahren zur Desinfektion von Abfällen, insbesondere klinischen Abfällen, bei dem die Abfälle in durch abnehmbare Behälterdeckel (62) dicht verschlossenen Abfallbehältern (9) einer Erhitzung ausgesetzt werden,
**dadurch gekennzeichnet,**
daß in einen oder gleichzeitig in mehrere Abfälle enthaltende und durch abnehmbare Behälterdeckel (62) keimdicht geschlossene Abfallbehälter (9) aus einem Dampfkessel (3) von unten überhitzter Wasserdampf von wenigstens 130°C zugeführt wird und daß diese Dampfzufuhr über eine vorwählbare Behandlungsdauer erfolgt, die erst zu laufen beginnt, wenn im oberen Bereich des Abfallbehälters bzw. aller Abfallbehälter (9) eine vorbestimmte Innentemperatur von wenigstens 110°C erreicht ist.

2. Verfahren nach Anspruch 1, dadurch gekennzeichnet, daß die Temperatur des injizierten Wasserdampfes zwischen 130°C und 200°C beträgt.

3. Verfahren nach Anspruch 1 oder 2, dadurch gekennzeichnet, daß in dem bzw. den Abfallbehältern (9) während der Dampfinjektion ein Überdruck von etwa 0,5 bis 0,8 bar herrscht.

4. Verfahren nach einem der Ansprüche 1 bis 3, dadurch gekennzeichnet, daß die vom injizierten Wasserdampf verdrängten Gasinhalte des bzw. der Abfallbehälter (9) aus dem jeweils oberen Bereich der Abfallbehälter (9) in geschlossenem Kreislauf über einen Kondensator (86) in den Dampfkessel (3) zurückgeführt werden.

5. Vorrichtung zur Durchführung des Verfahrens nach einem der Ansprüche 1 bis 4, dadurch gekennzeichnet, daß in einem von thermischen Schutzwänden (10 bis 13) und einem Boden (15) umschlossenen Behandlungsraum (2, 14) angeordneten, eine horizontale Abstellfläche bildenden Abstellrahmen (17) zum Anschließen eines oder mehrerer Abfallbehälter (9) rasterartig mehrere Dampfinjektoren ( 24) angeordnet sind, welche gemeinsam über ein Ventil (29) mit einem Dampfkessel (3) verbunden und einzeln mit Injektorventilen (53) der Abfallbehälter (9) kuppelbar sind und daß Auslaßrohrstutzen (111) vorgesehen sind, welche die Behälterdeckel (62) durchdringen und über eine Sammelleitung (85) gemeinsam an einen Dampfkondensator (86) angeschlossen sind, von dem das anfallende Kondensat in den Dampfkessel (3) geleitet wird, und daß elektrische Temperaturmelder (120), die jeweils durch die Behälterdeckel (62) in die einzelnen Abfallbehälter (9) ragen, bei Erreichen einer voreinstellbaren Betriebstemperatur ein Startsignal für eine wählbare Betriebsdauer erzeugen.

6. Vorrichtung nach Anspruch 5, dadurch gekennzeichnet, daß die Behälterdeckel (62) jeweils Öffnungen (73) aufweisen, die mittels durchstoßbarer oder entfernbarer Verschlußstopfen (74) verschlossen sind und durch welche die Auslaßrohrstutzen (111) und die Temperaturmelder (120) in die einzelnen Abfallbehälter (9) ragen.

7. Vorrichtung nach Anspruch 6, dadurch gekennzeichnet, daß die jeweils einzelnen Abfallbehältern (9) zugeordneten Auslaßrohrstutzen (111) zusammen mit Dichtungselementen (112) rasterartig in einem Rasterrahmen (98) angeordnet und gemeinsam in die Öffnungen (73) der Behälterdeckel (62) einführbar sind.

8. Vorrichtung nach einem der Ansprüche 5 bis 7, dadurch gekennzeichnet, daß die Temperaturmelder 120) mit Schutzrohren 121) versehen und die Auslaßrohrstutzen (111) axial durchragend im Rasterrahmen (98) befestigt sind.

9. Vorrichtung nach einem der Ansprüche 6 bis 8, dadurch gekennzeichnet, daß der Rasterrahmen (98) aus miteinander und mit der Sammelleitung (85) verbundenen Rohren besteht, in welchen die Auslaßrohrstutzen (111) offen enden.

10. Vorrichtung nach einem der Ansprüche 5 bis 9, dadurch gekennzeichnet, daß der Rasterrahmen (98) um eine horizontale Schwenkachse (108) aus seiner horizontalen Betriebsposition in eine etwa vertikale Ruheposition schwenkbar an einem mit dem Abstellrahmen (17) in verbundenen Aufnahmerahmen (18) angelenkt ist.

11. Vorrichtung nach einem der Ansprüche 5 bis 10, dadurch gekennzeichnet, daß die Verschlußstopfen (74) aus gummi-elastischem Material bestehen und jeweils eine den Rand einer Öffnung (73) des Behälterdeckels (62) mit Klemmsitz aufnehmende Ringnut (76) sowie einen am Öffnungsrand anliegenden Ringflansch (75) aufweisen.

12. Vorrichtung nach einem der Ansprüche 5 bis 11, dadurch gekennzeichnet, daß in einem vertikalen Abstand oberhalb des Abstellrahmens (17) der Aufnahmerahmen (18) für die rasterorientierte Aufnahme der einzelnen Abfallbehälter (9) angeordnet ist, der gleich viele von Rahmenschenkeln umschlossene Aufnahmefelder aufweist wie im Abstellrahmen Injektorventile (53) vorhanden sind.

13. Vorrichtung nach einem der Ansprüche 5 bis 12, dadurch gekennzeichnet, daß auf dem Abstellrahmen (17) jeweils konzentrisch zu den einzelnen Dampfinjektoren (24) Zentrierelemente (48), vorzugsweise in Form konischer Ringe oder keilförmiger Finger, für die unteren Ränder der einzelnen Abfallbehälter (9) angeordnet sind.

14. Vorrichtung nach einem der Ansprüche 5 bis 13, dadurch gekennzeichnet, daß die Dampfinjektoren (24) aus vertikalen Flanschhülsen (39) mit radialen Dichtungsringschultern (45) bestehen und
daß die Injektorventile (53) jeweils aus Rohrstutzen (54) bestehen, welche zentral im Behälterboden (52) angeordnet und mittels einer durch das in seine Bohrung (56) einführbare Rohrstück (40) nach oben wegstoßbare Verschlußkappe (55) keimdicht verschlossen sind.

15. Vorrichtung nach einem der Ansprüche 5 bis 14, dadurch gekennzeichnet, daß der Abstellrahmen (17) aus Rohren besteht, deren Hohlräume nach außen abgedichtet sind und nur mit den Dampfinjektoren (24) und mit einer Zufuhrleitung (28) des Dampfkessels (39 in Verbindung stehen.

16. Vorrichtung nach einem der Ansprüche 5 bis 15, dadurch gekennzeichnet, daß der Behandlungsraum (14) mit dem die Dampfinjektoren (24) aufweisenden Abstellrahmen (17), der Dampfkessel (3) mit dem Dampfkondensator (86), den zugehörigen Verbindungsleitungen (28, 85, 87, 89, 93) und einem Brennstoffbehälter (97) sowie mit einer elektrischen Schalt- und Steuerungseinheit (4) gemeinsam als Gesamtanlage auf einer transportablen Konsole (1) angeordnet sind.

17. Vorrichtung nach Anspruch 16, dadurch gekennzeichnet, daß auf der Konsole (1) neben dem Behandlungsraum (14) ein Kran (5) zum Einsetzen und Herausheben der Abfallbehälter (9) angeordnet ist.

18. Vorrichtung nach Anspruch 17, dadurch gekennzeichnet, daß im Arbeitsbereich des Krans auf der Konsole (1) eine Müllpresse (6) angeordnet ist.
